# EUROPEAN PATENT APPLICATION

(11) **EP 0 655 457 A1**
(43) Date of publication of application: **31.05.1995**
(21) Application number: 94908194.7
(22) Date of filing: 04.08.1993
(51) Int. Cl.: C07H 15/26, C07H 19/01, A61K 31/70

(54) **MORANOLINE DERIVATIVE**

(30) Priority: 14.08.1992 JP 238885/92; 12.03.1993 JP 77499/93
(71) Applicant: NIPPON SHINYAKU COMPANY, LIMITED, Minami-ku Kyoto-shi Kyoto 601 (JP); HASEGAWA, Akira, Gifu-shi, Gifu-ken 501-31 (JP)
(72) Inventor: HASEGAWA, Akira, Gifu-shi, Gifu-ken 501-31 (JP); KISO, Makoto, Motosugun, Gifu 501-12 (JP); YOSHIKUNI, Yoshiaki, Uji-shi, Kyoto 611 (JP)
(74) Representative: Alber, Norbert
(86) International application number: JP9301099
(87) International publication number: WO9404546

(57) **Abstract**

A novel sialic acid derivative containing moranoline, represented by general formula (I), which has a cell fusion inhibitor activity and is useful as a medicine for preventing or treating inflammation and accompanying thrombosis, rheumatism, immuno-logical diseases, viral infections and cancer, wherein R¹ represents hydrogen or lower alkyl and R² represents hydroxy or lower alkoxy, or alternatively R¹ and R² may be combined together to represent a single bond; and R³s may be the same or different from one another and each represents hydrogen or acetyl.

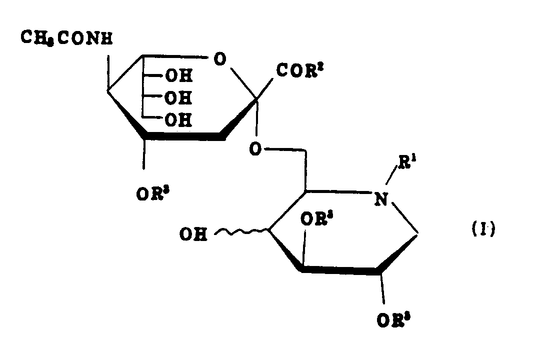

## Description

### TECHNICAL FIELD

The present invention relates to novel moranoline derivatives of the following general formula [I]. The compounds of the present invention are useful in the medical field, for example as an antiinflammatory agent or an antiviral agent. 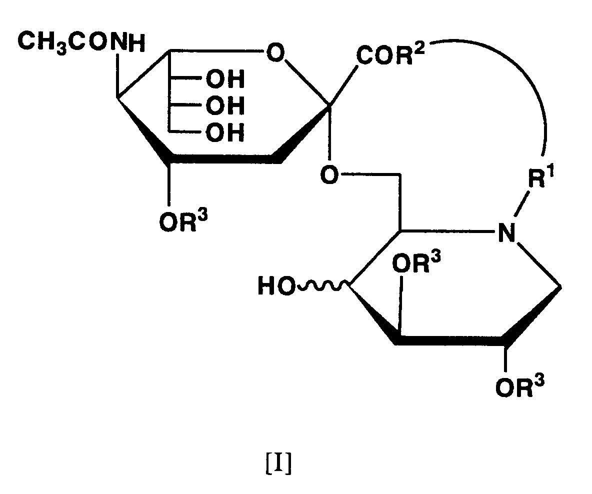
wherein R¹ represents hydrogen or lower alkyl; R² represents hydroxy or lower alkoxy, or R¹ and R², taken together, represent a single bond; R³ may be the same or different and each represents hydrogen or acetyl.

### BACKGROUND ART

Recent research has revealed that the carbohydrate moieties of glycolipids and glycoproteins containing a sialic acid have receptor functions for hormones, bacterial toxins, viruses, etc. and are profoundly concerned in fundamental, dynamic vital phenomena such as cognition, differentiation, growth, adhesion, canceration, immunity and aging of cells.

Therefore, sialic acid derivatives are attracting attention as substances having useful physiological activities and are being explored for potential application to the therapy, diagnosis and prevention of diseases. Such antiviral agents are disclosed in Japanese Kokai Tokkyo Koho 63-139193 and 03-251593 and the like, and immunomodulators are described in Japanese Kokai Tokkyo Koho 61-243074 and 62-209094. Furthermore, the application of such derivatives to cancer diagnosis and therapy is described in Japanese Kokai Tokkyo Koho 61-63700, for instance.

The inventors of this invention explored into a variety of carbohydrate antigen derivatives and discovering novel carbohydrate antigen derivatives inclusive of certain moranolines, which have excellent pharmacologic activities, already applied for patent (Japanese Application No. 04-46081).

However, since these sialic acid derivatives occur only in trace amounts in the natural kingdom, consider able difficulties are encountered in obtaining them as pure simple compounds from a living body. It is for this reason that research into sialic acid derivatives as drugs has only just begun in these recent few years, although great hopes are harbored for their clinical application.

### DISCLOSURE OF INVENTION

The object of this invention is to provide a moranoline derivative which is useful as a medicine and is novel in chemical structure.

The inventors continued research with increased enthusiasm and found that compounds of general formula [I], which are sialic acid derivatives of glucose type and galactose type moranolines, are suitable for the above-mentioned object. This invention has been developed on the basis of the above finding.

The essence of this invention lies in the very structure of the compounds of general formula [I]. The compounds of this invention are not only a novel compounds but have very excellent pharmacological activities as will be described later.

Referring to general formula [I], the lower alkyl R¹ is a straight-chain or branched alkyl group of preferably 1-7 carbon atoms, such as methyl, ethyl, n-propyl, isopropyl, n-butyl, isobutyl, t-butyl, n-pentyl, isopentyl, n-hexyl, isohexyl, n-heptyl and isoheptyl.

Referring, further, to general formula [I], the lower alkoxy R² is a straight-chain or branched alkoxy group which, as it is the case with R¹, is preferably a group of 1-7 carbon atoms.

As examples of the compounds of this invention, the following compounds can be mentioned in addition to the compounds mentioned in the examples of production which appear hereinafter. It should be understood that this invention is not limited to these compounds but encompases other compounds as well.

O-(Ethyl 5-acetamido-3,5-dideoxy-D-glycero-α-D-galacto-2-nonulopyranosylonate)-(2→6)-1,5-dideoxy-1,5-imino-D-glucitol
O-(Propyl 5-acetamido-3,5-dideoxy-D-glycero-α-D-galacto-2-nonulopyranosylonate)-(2→6)-1,5-dideoxy-1,5-imino-D-glucitol
O-(Butyl 5-acetamido-3,5-dideoxy-D-glycero-α-D-galacto-2-nonulopyranosylonate)-(2→6)-1,5-dideoxy-1,5-imino-D-glucitol
O-(Pentyl 5-acetamido-3,5-dideoxy-D-glycero-α-D-galacto-2-nonulopyranosylonate)-(2→6)-1,5-dideoxy-1,5-imino-D-glucitol
O-(Hexyl 5-acetamido-3,5-dideoxy-D-glycer-α-D-galacto-2-nonulopyranosylonate)-(2→6)-1,5-dideoxy-1,5-imino-D-glucitol
O-(Heptyl 5-acetamido-3,5-dideoxy-D-glycero-α-D-galacto-2-nonulopyranosylonate)-(2→6)-1,5-dideoxy-1,5-imino-D-glucitol
O-(5-Acetamido-3,5-dideoxy-D-glycero-α-D-galacto-2-nonulopyranosylonic acid)-(2→6)-1,5-dideoxy-N-methyl-1,5-imino-D-glucitol
O-(5-Acetamido-3,5-dideoxy-D-glycero-α-D-glacto-2-nonulopyranosylonic acid)-(2→6)-1,5-dideoxy-N-ethyl-1,5-imino-D-glucitol
O-(5-Acetamido-3,5-dideoxy-D-glycero-α-D-glacto-2-nonulopyranosylonic acid)-(2→6)-1,5-dideoxy-N-propyl-1,5-imino-D-glucitol
O-(5-Acetamido-3,5-dideoxy-D-glycero-α-D-galacto-2-nonulopyranosylonic acid)-(2→6)-1,5-dideoxy-N-butyl-1,5-imino-D-glucitol
O-(5-Acetamido-3,5-dideoxy-D-glycero-α-D-galacto-2-nonulopyranosylonic acid)-(2→6)-1,5-dideoxy-N-pentyl-1,5-imino-D-glucitol
O-(5-Acetamido-3,5-dideoxy-D-glycero-α-D-galacto-2-nonulopyranosylonic acid)-(2→6)-1,5-dideoxy-N-hexyl-1,5-imino-D-glucitol
O-(5-Acetamido-3,5-dideoxy-D-glycero-α-D-galacto-2-nonulopyranosylonic acid)-(2→6)-1,5-dideoxy-N-heptyl-1,5-imino-D-glucitol
O-(Heptyl 5-acetamido-3,5-dideoxy-D-glycero-α-D-galacto-2-nonulopyranosylonate)-(2→6)-1,5-dideoxy-N-methyl-1,5-imino-D-glucitol
O-(Pentyl 5-acetamido-3,5-dideoxy-D-glycero-α-D-galacto-2-nonulopyranosylonate)-(2→6)-1,5-dideoxy-N-propyl-1,5-imino-D-glucitol
O-(Propyl 5-acetamido-3,5-dideoxy-D-glycero-α-D-galacto-2-nonulopyranosylonate)-(2→6)-1,5-dideoxy-N-pentyl-1,5-imino-D-glucitol
O-(Metyl 5-acetamido-3,5-dideoxy-D-glycero-α-D-galacto-2-nonulopyranosylonate)-(2→6)-1,5-dideoxy-N-heptyl-1,5-imino-D-glucitol
O-(Ethyl 5-acetamido-3,5-dideoxy-D-glycero-α-D-galacto-2-nonulopyranosylonate)-(2→6)-1,5-dideoxy-1,5-imino-D-galactitol
O-(Propyl 5-acetamido-3,5-dideoxy-D-glycero-α-D-galacto-2-nonulopyranosylonate)-(2→6)-1,5-dideoxy-1,5-imino-D-galactitol
O-(Butyl 5-acetamido-3,5-dideoxy-D-glycero-α-D-galacto-2-nonulopyranosylonate)-(2→6)-1,5-dideoxy-1,5-imino-D-galactitol
O-(Pentyl 5-acetamido-3,5-dideoxy-D-glycero-α-D-galacto-2-nonulopyranosylonate)-(2→6)-1,5-dideoxy-1,5-imino-D-galactitol
O-(Hexyl 5-acetamido-3,5-dideoxy-D-glycero-α-D-galacto-2-nonulopyranosylonate)-(2→6)-1,5-dideoxy-1,5-imino-D-galactitol
O-(Heptyl 5-acetamido-3,5-dideoxy-D-glycero-α-D-galacto-2-nonulopyranosylonate)-(2→6)-1,5-dideoxy-1,5-imino-D-galactitol
O-(5-Acetamido-3,5-dideoxy-D-glycero-α-D-galacto-2-nonulopyranosylonic acid)-(2→6)-1,5-dideoxy-N-methyl-1,5-imino-D-galactitol
O-(5-Acetamido-3,5-dideoxy-D-glycero-α-D-galacto-2-nonulopyranosylonic acid)-(2→6)-1,5-dideoxy-N-ethyl-1,5-imino-D-galactitol
O-(5-Acetamido-3,5-dideoxy-D-glycero-α-D-galacto-2-nonulopyranosylonic acid)-(2→6)-1,5-dideoxy-N-propyl-1,5-imino-D-galactitol
O-(5-Acetamido-3,5-dideoxy-D-glycero-α-D-galacto-2-nonulopyranosylonic acid)-(2→6)-1,5-dideoxy-N-butyl-1,5-imino-D-galactitol
O-(5-Acetamido-3,5-dideoxy-D-glycero-α-D-galacto-2-nonulopyranosylonic acid)-(2→6)-1,5-dideoxy-N-pentyl-1,5-imino-D-galactitol
O-(5-Acetamido-3,5-dideoxy-D-glycero-α-D-galacto-2-nonulopyranosylonic acid)-(2→6)-1,5-dideoxy-N-hexyl-1,5-imino-D-galactitol
O-(5-Acetamido-3,5-dideoxy-D-glycero-α-D-galacto-2-nonulopyranosylonic acid)-(2→6)-1,5-dideoxy-N-heptyl-1,5-imino-D-galactitol
O-(Heptyl 5-acetamido-3,5-dideoxy-D-glycero-α-D-galacto-2-nonulopyranosylonate)-(2→6)-1,5-dideoxy-N-methyl-1,5-imino-D-galactitol
O-(Pentyl 5-acetamido-3,5-dideoxy-D-glycero-α-D-galacto-2-nonulopyranosylonate)-(2→6)-1,5-dideoxy-N-propyl-1,5-imino-D-galactitol
O-(Propyl 5-acetamido-3,5-dideoxy-D-glycero-α-D-galacto-2-nonulopyranosylonate)-(2→6)-1,5-dideoxy-N-pentyl-1,5-imino-D-galactitol
O-(Methyl 5-acetamido-3,5-dideoxy-D-glycero-α-D-galacto-2-nonulopyranosylonate)-(2→6)-1,5-dideoxy-N-heptyl-1,5-imino-D-galactitol
As will be described in detail in the test examples presented hereinafter, the compounds of this invention has sialidase inhibitory and cell adhesion-inhibiting activities. Thus being capable of inhibiting viral and human sialidases, the compounds of this invention are certain to be useful as an antiviral agent or a drug which contributes to the increase of duration of various sialic acid-containing glycoproteins in the blood.

The compounds of the present invention having cell adhesion-inhibitory activity inhibit secretin antagonistically which is present in endothelial cells, thereby inhibit adhesion of leukocytes or cancer cells to endothelial cells so that it is certain to be useful in the prevention and treatment of inflammation and associated thrombosis, rheumatism, infectious diseases, immune diseases, AIDS and cancer.

Therefore, the compounds of this invention is of use as an antiviral agent, antiinflammatory agent, antithrombotic agent, antirheumatic agent, antiinfective agent, anti-AIDS agent, or a prophylactic and therapeutic drug for immune diseases and cancer.

### BEST MODE OF CARRYING OUT THE INVENTION

The following example and test examples are intended to illustrate this invention in further detail.

### [Example]

### Preparation of sialylmoranoline derivatives(1) Synthesis of 2,3-di-O-acetyl-4,6-O-benzylidene-N-benzyloxycarbonyl-1,5-dideoxy-1,5-imino-D-glucitol

In pyridine (10 ml) was dissolved 4,6-O-benzylidene-N-benzyloxycarbonyl-1,5-dideoxy-1,5-imino-D-glucitol (3.04 g) of the following formula followed by addition of acetic anhydride (5 ml) and the mixture was stirred at room temperature overnight. 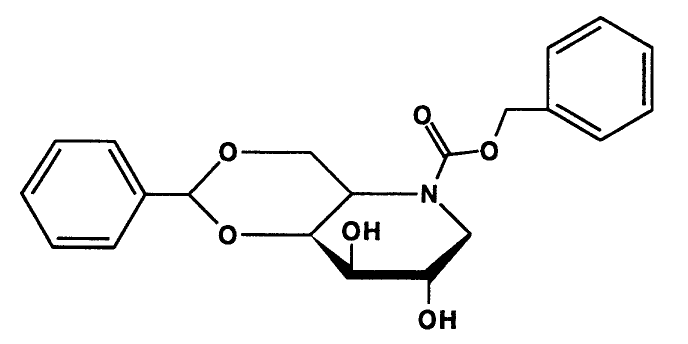
After the completion of reaction was confirmed by TLC, methanol (20 ml) was added at 0°C and the mixture was stirred for 30 minutes. This reaction mixture was concentrated under reduced pressure and the resulting residue was extracted with dichloromethane. The extract was washed with 2N-hydrochloric acid and water, dried over sodium sulfate, filtered and washed with dichloromethane thoroughly. The filtrate and the washings were combined followed by concentration under reduced pressure to provide the title compound (1) (3.79 g, quantitatively).

### Physical constants

1. Specific rotation [α]D²⁵-18.68° (C=0.974, dichloroethane)
2. Elemental analysis [C₂₅H₂₇O₈N]

| | | | |
|---|---|---|---|
| Calcd. | C=63.96% | H=5.80% | N=2.98% |
| Found | C=63.70% | H=5.76% | N=3.27% |

### (2) Synthesis of 2,3-di-O-acetyl-N-benzyloxycarbonyl-1,5-dideoxy-1,5-imino-D-glucitol

Compound (1) (720 mg) was dissolved in 80% aqueous solution of acetic acid (20 ml) and the solution was stirred at 45°C overnight. After the completion of reaction was confirmed by TLC, the reaction mixture was concentrated under reduced pressure and the resulting residue was subjected to column chromatography (Wakogel C-200, eluent: ethyl acetate-hexane, 3:1) to give Compound (2) (580 mg, 79%).

### Physical constants

1. Specific rotation [α]D²⁵-1.74° (C=1.146, dichloroethane)
2. Elemental analysis [C₁₈H₂₃O₈N]

| | | | |
|---|---|---|---|
| Calcd. | C=56.69% | H=6.08% | N=3.67% |
| Found | C=56.49% | H=5.80% | N=3.69% |

### (3) Synthesis of 2,3-di-O-acetyl-N-benzyloxycarbonyl-6-O-tert-butyldiphenylsilyl-1,5-dideoxy-1,5-imino-D-glucitol

In pyridine (25 ml) was dissolved Compound (2) (1.31 g) and after cooling to 0°C, tert-butyldiphenylsilyl chloride (2.7 ml, 3 equivalents) was added. The mixture was stirred at 0°C-20°C overnight. After the completion of reaction was confirmed by TLC, the reaction mixture was extracted with dichloromethane and the extract was washed with 2N-hydrochloric acid and water. The extract was dried over sodium sulfate, filtered and thoroughly washed with dichloromethane. The filtrate and the washings were combined followed by concentration under reduced pressure and the resuling residue was subjected to column chromatography (Wakogel C-200, eluent: ethyl acetate-hexane, 1:2) to provide Compound (3) (1.98 g, 93%).

### Physical constants

1. Specific rotation [α]D²⁵+2.55° (C=1.094, dichloroethane)
2. Elemental analysis [C₃₄H₄₁O₈NSi]

| | | | |
|---|---|---|---|
| Calcd. | C=65.89% | H=6.67% | N=2.26% |
| Found | C=65.98% | H=6.54% | N=2.25% |

### (4) Synthesis of 2,3,4-tri-O-acetyl-N-benzyloxycarbonyl-6-O-tert-butyldiphenylsilyl-1,5-dideoxy-1,5-imino-D-galactitol

In a mixture of dichloromethane (30 ml) and pyridine (15 ml) was dissolved Compound (3) (1.05 g) and after cooling to -15°C, trifluoromethanesulfonic anhydride/dichloromethane (0.58 ml, 2 equivalents/10 ml) was added dropwise. The mixture was stirred at -15°C for 3 hours. The completion of reaction was confirmed by TLC. The reaction mixture was neutralized with triethylamine and extracted with dichloromethane. The extract was washed with 2N-hydrochloric acid and water. The washed extract was dried over sodium sulfate and filtered. The filtrate and the washings were combined followed by concentration under reduced pressure. The resuling residue was dissolved in acetonitrile (40 ml) and after addition of the desiccant molecular sieve 3A (120 mg), the solution was stirred for 1 hour. Thereafter, cesium acetate (1.63 g, 5 equivalents) and 1,4,7,10,13,16-hexaoxacyclooctadecane (0.67 g, 1.5 equivalents) were added and the mixture was stirred at room temperature overnight. After the completion of reaction was confirmed by TLC, the molecular sieve was filtered off and washed well with dichloromethane. The filtrate and the washings were combined followed by concentration under reduced pressure and the resuling residue was subjected to column chromatography (Wakogel C-200, eluent: ethyl acetate-hexane, 1:5) to provide Compound (4) (0.75 g, 67%).

### Physical constants

1. Specific rotation [α]D²⁵+0.091° (C=0.880, dichloroethane)
2. Elemental analysis [C₃₆H₄₃O₉NSi]

| | | | |
|---|---|---|---|
| Calcd. | C=65.33% | H=6.55% | N=2.12% |
| Found | C=65.34% | H=6.66% | N=2.01% |

### (5) Synthesis of 2,3,4-tri-O-acetyl-N-benzyloxycarbonyl-1,5-dideoxy-1,5-imino-D-galactitol

In dichloromethane (40 ml) was dissolved Compound (4) (630 mg), followed by addition of boron trifluoride diethyl ether complex (7 ml) at 0°C. The mixture was then stirred at room temperature for 10 hours. After the completion of reaction was confirmed by TLC, the reaction mixture was extracted with dichloromethane and washed with aqueous sodium carbonate and water. The extract was dried over sodium sulfate, filtered and washed with dichloromethane. The filtrate and the washings were combined followed by concentration under reduced pressure and the resuling residue was subjected to column chromatography (Wakogel C-300, eluent: dichloromethane-methanol, 200:1) to provide Compound (5) (324 mg, 81%).

### Physical constants

1. Specific rotation [α]D²⁵+20.00° (C=1.550, dichloroethane)
2. Elemental analysis [C₂₀H₂₅O₉N]

| | | | |
|---|---|---|---|
| Calcd. | C=56.73% | H=5.95% | N=3.31% |
| Found | C=56.63% | H=6.11% | N=3.32% |

### (6) Synthesis of O-(methyl 5-acetamido-4,7,8,9-tetra-O-acetyl-3,5-dideoxy-D-glycero-α-D-galacto-2-nonulopyranosylonate)-(2→6)-2,3-di-O-acetyl-N-benzyloxycarbonyl-1,5-dideoxy-1,5-imino-D-glucitol

In acetonitrile (10 ml) were dissolved Compound (2) (120 mg) and 2-methylthio-sialic acid derivative of the following formula (α:β=1:1) (328 mg, 2 equivalents), followed by addition of the desiccant molecular sieve 3A (500 mg), and the mixture was stirred overnight. 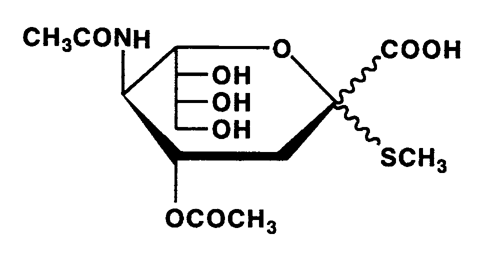
After cooling to -15°C, dimethyl(methylthio)sulfonium triflate (435 mg, 6 equivalents) was added and the mixture was stirred at 0°C overnight. After the completion of reaction was comfirmed by TLC, the molecular sieves were filtered off and the filtrate was extracted with dichloromethane and washed with aqueous sodium carbonate and water. The extract was dried over sodium sulfate and filtered. The filtrate and the washings were combined followed by concentration under reduced pressure. The resuling residue was subjected to column chromatography (Wakogel C-300, eluent: dichloroethane-methanol=70:1) to provide Compound (6) (167 mg, 62%).

### Physical constants

1. Specific rotation [α]D²⁵-15.95° (C=0.840, dichloroethane)
2. Elemental analysis [C₃₈H₅₀O₂₀N₂]

| | | | |
|---|---|---|---|
| Calcd. | C=53.39% | H=5.90% | N=3.28% |
| Found | C=53.52% | H=5.85% | N=3.18% |

### (7) Synthesis of O-(methyl 5-acetamido-4,7,8,9-tetra-O-acetyl-3,5-dideoxy-D-glycero-α-D-galacto-2-nonulopyranosylonate)-(2→6)-2,3,4-tri-O-acetyl-N-benzyloxycarbonyl-1,5-dideoxy-1,5-imino-D-galactitol

In acetonitrile (15 ml) were dissolved Compound (5) (147 mg) and said 2-methylthio-sialic acid derivative (a:b=1:1) (308 mg, 1.7 equivalents) followed by addition of the desiccant molecular sieves 3A (500 mg), and the mixture was stirred at room temperature overnight. After the mixture was cooled to -40°C, N-iodosuccinimide (266 mg, 3.4 equivalents) and trifluoromethanesulfonic acid (10ml, 0.34 equivalents) were added and the mixture was stirred at -40°C overnight. After the completion of reaction was confirmed by TLC, the molecular sieves were filtered off, extracted with dichloromethane, and washed with aqueous sodium carbonate, aqueous sodium thiosulfate and water. The extract was dried over sodium sulfate and filtered and the filtrate and the washings were combined followed by concentration under reduced pressure. The resuling residue was subjected to column chromatography (Wakogel C-200, eluent: toluene-methanol=50:1) to provide Compound (7) (174 mg, 56%).

### Physical constants

1. Specific rotation [α]D²⁵+24.16° (C=0.240, dichloromethane)
2. Elemental analysis [C₄₀H₅₂O₂₁N₂]

| | | | |
|---|---|---|---|
| Calcd. | C=53.57% | H=5.84% | N=3.12% |
| Found | C=53.76% | H=5.68% | N=2.98% |

### Synthesis of (8) 6-O-(5-acetamido-3,5-dideoxy-D-glycero-α-D-galacto-2-nonulopyranosylono-1',5-N-lactam)-1,5-dideoxy-1,5-imino-D-glucitol and (9) O-(5-acetamido-3,5-dideoxy-D-glycero-α-D-galacto-2-nonulopyranosylonic acid)-(2→6)-1,5-dideoxy-N-methyl-1,5-imino-D-glucitol

In methanol (15 ml) was dissolved Compound (6) (390 mg) followed by addition of methanol-washed 10% palladium-on-carbon (400 mg) and direct hydrogenation reaction was carried out at room temperature with stirring for 30 minutes. After the completion of reaction was confirmed by TLC, the 10% palladium-on-carbon was filtered off and washed with methanol. The filtrate and the washings were combined followed by concentration under reduced pressure. The resuling residue was dissolved in methanol (20 ml) and after addition of sodium methoxide to bring the mixture to pH≒12, the whole mixture was stirred at room temperature overnight. Then, 0.2N-aqueous potassium hydroxide solution (4 ml) was added and the mixture was further stirred at room temperature overnight. After the completion of reaction was confirmed by TLC, the reaction mixture was neutralized with the ion exchange resin Amberlite IR-120 (H+). The resin was filtered off and washed thoroughly with methanol and water. The filtrate and the washings were combined followed by concentration under reduced pressure and the resuling residue was subjected to gel filtration (Sephadex LH-20) to provide Compound (8) (161 mg, 81%) and Compound (9) (41 mg, 19%). Physical constants of Compound (8)
1. Specific rotation [α]D²⁵+38.01° (C=0.826, methanol)
2. Elemental analysis [C₁₇H₂₈O₁₁N₂]

| | | | |
|---|---|---|---|
| Calcd. | C=46.79% | H=6.47% | N=6.42% |
| Found | C=46.65% | H=6.64% | N=6.24% |

### Physical constants of Compound (9)

1. Specific rotation [α]D²⁵+9.92° (C=0.524, water-ethanol=2:3)
2. Elemental analysis [C₁₈H₃₂O₁₂N₂]

| | | | |
|---|---|---|---|
| Calcd. | C=46.15% | H=6.89% | N=5.98% |
| Found | C=46.01% | H=7.06% | N=5.80% |

### Synthesis of (10) 6-O-(5-acetamido-3,5-dideoxy-D-glycero-α-D-galacto-2-nonulopyranosylono-1',5-N-lactam)-1,5-dideoxy-1,5-imino-D-galactitol and(11) O-(5-acetamido-3,5-dideoxy-D-glycero-α-D-galacto-2-nonulopyranosylonic acid)-(2→6)-1,5-dideoxy-N-methyl-1,5-imino-D-galactitol

In methanol (15 ml) was dissolved Compound (7) (174 mg) followed by addition of methanol-washed 10% palladium-on-carbon (200 mg). Direct hydrogenation was performed at room temperature with stirring for 30 minutes. After the completion of reaction was confirmed by TLC, the palladium-on-carbon was filtered off and washed with methanol. The filtrate and washings were combined followed by concentration under reduced pressure. The resuling residue was dissolved in methanol (20 ml) and after addition of sodium methoxide to adjust the mixture to pH=12, the mixture was stirred at room temperature overnight. Then, 0.2N-aqueous potassium hydroxide solution (4 ml) was added and the mixture was stirred at room temperature overnight. After the completion of reaction was confirmed by TLC, the reaction mixture was neutralized with the ion exchange resin Amberlite IR-120 (H+) and the resin was filtered off and washed thoroughly with methanol and water. The filtrate and the washings were combined followed by concentration under reduced pressure and the resuling residue was subjected to gel filtration (Sephadex LH-20) to provide Compound (10) (74 mg, 87%) and Compound (11) (11 mg, 13%).

### Physical constants of Compound (10)

1. Specific rotation [α]D²⁵+36.12° (C=1.434, methanol)
2. Elemental analysis [C₁₇H₂₈O₁₁N₂]

| | | | |
|---|---|---|---|
| Calcd. | C=46.79% | H=6.47% | N=6.42% |
| Found | C=46.76% | H=6.17% | N=6.34% |

### Physical constants of Compound (11)

1. Specific rotation [α]D²⁵+11.34° (C=0.194, water-ethanol=2:3)
2. Elemental analysis [C₁₈H₃₂O₁₂N₂]

| | | | |
|---|---|---|---|
| Calcd. | C=46.15% | H=6.89% | N=5.98% |
| Found | C=45.90% | H=6.85% | N=6.27% |

### [Test Example]

The effect of the synthetic sugar on the binding of activated human vascular endothelial cells and cultured human leukemia HL60 cells

### [Cell culture]

Cultured human vascular endothelial cells were separated from the human umbilical vein by collagenase treatment and cultured in a culture flask coated with 1% gelatin or fibronectin at a concentration of 0.02 mg/cm². The growth medium used was prepared by adding 15% fetal calf serum (FCS), 45 mg/l ECGS, 90 mg/l heparin and 40 mg/l gentamicin to medium 199. The proliferation of the cultured human leukemia cell line HL60 was maintained using 10% FCS-containing RPMI-1640. Both cells were cultured in a CO₂ incubator (5% CO₂, 95% air) at 37°C.

### [Determination of cell adhesion]

The cultured human umbilical vein endothelial cells (HUVEC) were seeded on a 96-well microtiter plate which had been precoated with 0.1% gelatin and were incubated until the growth became confluent. After the cells were washed with 10% FCS-containing Dulbecco's modified Eagle's medium (DMEM), 100 µl of 10% FCS-DMEM containing recombinant human interleukin-1β (IL-1β) was added. The cells were then cultured in a CO₂ incubator at 37°C for 4 hours (activation). The HL60 cells were washed twice with serum-free DMEM, suspended in 0.5% glutaldehyde-containing Hanks' solution and fixed on wet ice for 30 minutes.

The fixed cells were washed twice with serum-free DMEM to remove the fixative. Then, 10% FCS-containing DMEM was added to the cells so as to provide a suspension of 2x10⁶ cells/ml, which was then preserved on wet ice till use.

The HUVEC activated above was washed with 10% FCS-containing DMEM and 50µl of 10% FCS-DMEM containing either anti-ELAM-1 antibody (BBA 2, British Biotechnology Ltd., Abinton) or the test substance was added followed by incubation at room temperature for 30 minutes. The fixed HL60 cells were added in 50 µl aliquots and the incubation was further continued at room temperature for 45 minutes.

After the unbound HL60 cells were washed off, the center of each well was photographed and the number of adhered cells in the photographing field was counted. With the number of HL60 cells bound to the IL-1β-activated HUVEC being taken as 100%, the effect of addition of the test substance was evaluated. The results are presented in Tables 1 and 2.

**Table 1**

| Inhibitory effect of moranoline derivatives on the ELAM-1 dependent adhesion of cultured human leukemia HL60 cells | | | |
|---|---|---|---|
| Treatment | Concentration (µg/ml) | n | Cell adhesion count (%) |
| Basal | | 5 | 28.2 (7.4) |
| Control | | 5 | 383.4 (100.0) |
| a-ELAM | 25 | 5 | 75.0 (19.6) |
| Example (9) | 100 | 5 | 188.4 (49.1) |
| Example (11) | 100 | 5 | 155.0 (40.4) |
| Basal stands for adhesion to non-activated HUVEC. Control stands for adhesion to HUVEC activated with IL-1β 10U/ml. α-ELAM stands for adhesion to activated HUVEC with addition of anti-ELAM-1 antibody. Example (9) and Example (11) stand for adhesion to activated HUVEC with addition of the corresponding compounds. | | | |

**Table 2**

| Inhibitory effect of moranoline derivatives on the ELAM-1 dependent adhesion of cultured human leukemia HL60 cells | | | |
|---|---|---|---|
| Treatment | Concentration (µg/ml) | n | Cell adhesion count (%) |
| Basal | | 6 | 77.7 (14.7) |
| Control | | 6 | 528.5 (100.0) |
| α-ELAM | 25 | 6 | 150.3 (28.4) |
| Example (8) | 50 | 6 | 473.7 (89.6) |
| Example (8) | 500 | 6 | 428.5 (81.1) |
| Example (10) | 50 | 6 | 494.7 (93.4) |
| Example (10) | 500 | 6 | 436.2 (82.5) |
| Basal stands for adhesion to non-activated HUVEC. Control stands for adhesion to HUVEC activated with IL-1β 10U/ml. α-ELAM stands for adhesion to activated HUVEC with addition of anti-ELAM-1 antibody. Example (8) and Example (10) stand for adhesion to activated HUVEC with addition of the corresponding compounds. | | | |

Referring to Table 1, it is clear that the compounds of Example (9) and Example (11), both of which are compounds of this invention, inhibit the adhesion of HL60 cells to activated HUVEC by 50.9% and 59.6%, respectively, at the concentration of 100 µg/ml, thus having inhibitory activity against ELAM-1-dependent cell adhesion.

Referring to Table 2, it is clear that the compounds of Example (8) and Example (10), both of which are compounds of this invention, inhibit the adhesion of HL60 cells to activated HUVEC by 18.9% and 17.5%, respectively, at the concentration of 500 µg/ml, thus having inhibitory activity against ELAM-1-dependent cell adhesion.

For use of the compound of this invention as a medicine, the compound is administered to animals inclusive of man, either as it is or in the form of a pharmaceutical composition containing 0.1% to 99.5%, preferably 0.5% to 90%, of the compound in a pharmaceutically acceptable, nontoxic and inert carrier.

The carrier that can be used includes solid, semi-solid or liquid diluents, fillers and other formulation auxiliaries and one or more of them can be used. The pharmaceutical composition is preferably administered in a unit dosage form. The pharmaceutical composition of this invention can be administered into tissues, topically (e.g. transdermally), or rectally. Of course, a dosage form suitable for each method of administration should be chosen. For example, administration into a tissue is particularly preferred.

The dosage for use as an antiviral drug is preferably selected with reference to patient factors, e.g. age and body weight, route of administration, type and severity of disease, etc. Generally, in terms of the daily amount of the active ingredient to be administered to an adult, the range of 100 mg-3 g/day/man, preferably 500 mg-1 g/day/man, is used. Depending on cases, a lower dose may be sufficient or a higher dose may be required. Preferably, the above-mentioned daily dose is administered in a single dose or in up to 3 divided doses.

## Claims

1. A moranoline derivative of the following general formula [I] 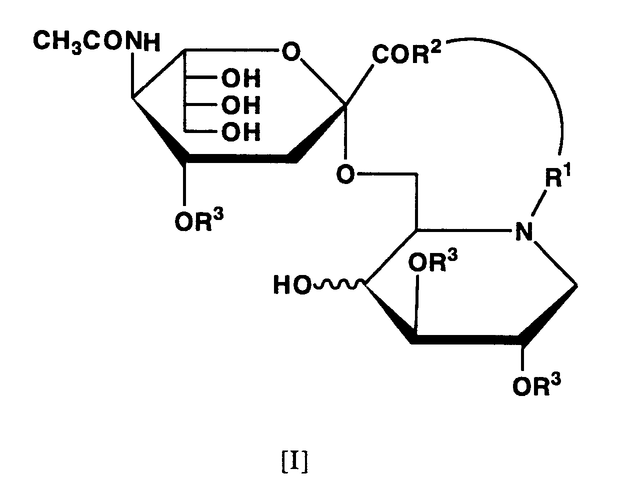 wherein R¹ represents hydrogen or lower alkyl; R² represents hydroxy or lower alkoxy, or R¹ and R², taken together, represent a single bond; R³ may be the same or different and each represents hydrogen or acetyl.

2. A moranoline derivative of the following formula

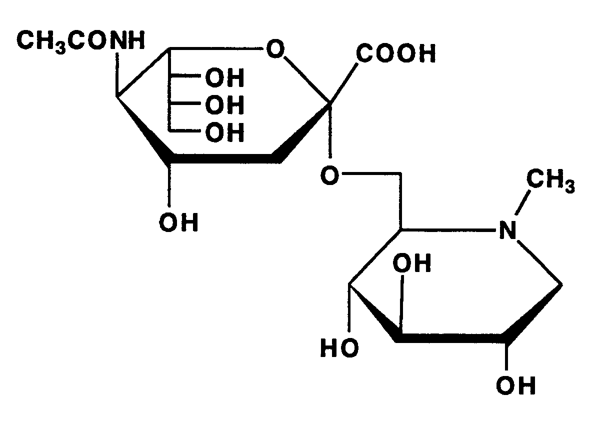

3. A moranoline derivative of the following formula

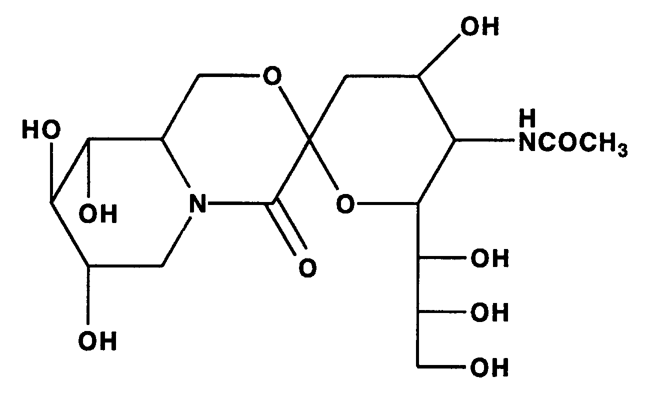

4. A moranoline derivative of the following formula

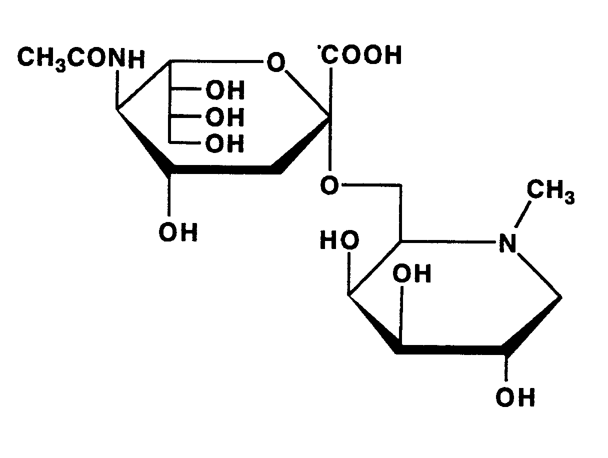

5. A moranoline derivative of the following formula
